# EUROPEAN PATENT SPECIFICATION

(11) **EP 3 600 303 B1**
(45) Date of publication and mention of the grant of the patent: **23.07.2025**
(21) Application number: 18846500.9
(22) Date of filing: 15.08.2018
(51) Int. Cl.: A61K 31/4164, A61K 9/06, A61K 9/10, A61K 9/19, A61K 9/20, A61K 9/28, A61K 9/48, A61K 31/437, A61K 38/14, A61P 25/00, A61K 45/06, A61K 9/00

(54) **A COMPOSITION COMPRISING A RIFAXIMIN AND A COMBINATION OF A VANCOMYCIN, A METRONIDAZOLE, AND/OR A TINIDAZOLE FOR TREATING AUTISM**
ZUSAMMENSETZUNG, UMFASSEND EIN RIFAXIMIN UND EINE KOMBINATION EINES VANCOMYCINS, EINES METRONIDAZOLS UND/ODER EINES TINIDAZOLS ZUR BEHANDLUNG VON AUTISMUS
COMPOSITION COMPRENANT UNE RIFAXIMINE ET UNE COMBINAISON D'UNE VANCOMYCINE, D'UN MÉTRONIDAZOLE ET/OU D'UN TINIDAZOLE POUR TRAITER L'AUTISME

(30) Priority: 15.08.2017 US 201762545989 P
(43) Date of publication of application: 05.02.2020
(73) Proprietor: Borody, Thomas Julius, Five Dock, NSW 2046 (AU)
(72) Inventor: Borody, Thomas Julius, Five Dock, NSW 2046 (AU)
(74) Representative: Zacco GmbH
(86) International application number: PCT/AU2018/000135
(87) International publication number: WO 2019/033142

(56) References cited:
- WO-A1-2011/050397
- WO-A1-2014/176632
- WO-A1-2019/040969
- BR-A2- 102015 000 951
- US-A1- 2004 170 617
- US-A1- 2012 207 726
- US-A1- 2012 252 775
- SANDLER RICHARD H ET AL: "Short-term benefit from oral vancomycin treatment of regressive-onset autism", JOURNAL OF CHILD NEUROLOGY, DECKER, CA, vol. 15, no. 7, 1 July 2000 (2000-07-01), pages 429 - 435, XP009093351, ISSN: 0883-0738
- O'MAHONY, S.M. ET AL.: "The Microbiome and Childhood Diseases: Focus on Brain- Gut Axis", BIRTH DEFECTS RESEARCH (PART C, vol. 105, 2015, pages 297 - 314, XP055570134

## Description

### Field

This invention generally relates to medicine and gastroenterology, pharmacology and microbiology. In alternative embodiments, provided are pharmaceutical compositions for use in treating, or preventing (acting as a prophylaxis) autism or an autism spectrum disorder (ASD), e.g., regressive autism. In alternative examples, these pharmaceutical compositions are dosaged and administered to children in need thereof. In alternative examples, pharmaceutical compositions are dosaged, formulated and dosaged as solid, liquid or aerosol preparations or formulations. In alternative embodiments, pharmaceutical compositions comprise rixafimin and other antimicrobial or antibiotic agent, for example, vancomycin, metronidazole, tinidazole or a combination thereof.

### Background

There are conditions which are clinically characterised by a super-infected stool, but where the bacterial super-infection can be difficult to detect these pose a medical challenge with treatment. Among such conditions are Irritable Bowel Syndrome, Colitis, and Autism/Autism Spectrum Disorder (ASD), to name a few. In ASD some researchers have identified unusual *Clostridia,* e.g. *Clostridium boltae* and *Desulfovibrios.* These may be an underlying pathogenic mechanism, and may cause the development and manufacture within the colonic flora of neurotoxins. Such neurotoxins can ultimately enter the peripheral circulation in a child that has acquired such an infection during a course of antibiotics in its youth - and the toxins attach themselves to various areas in the brain such as Broca's area, association areas in the parietal cortex and visual areas in the occipital cortex. When those parts of the brain are affected by the neurotoxins, neurotransmission is inhibited and the children develop the classic symptoms of Autism. This includes loss of learned speech, repetitive movements, an inability to see mother's eyes, or loss of eye contact. Given that the origin of the neurotoxins is the bowel flora - considered to be the body's largest organ albeit a 'virtual organ' since it does not carry human DNA cells - this super-infection can then become the target for therapy to reverse Autism. Sandler et al (Journal of Child Neurology; July 2000; 15, 7) reported that treatment of such children with vancomycin may suppress the production of these neurotoxins, resulting in reversal of behavioural changes and progress to normality within a few weeks of treatment on vancomycin, which is not absorbable. Other antibiotics can be used for this condition, for example, Rodakis reported that a child's autistic symptoms reversed almost completely when treated with an amoxycillin preparation (Rodakis J. Microbial Ecology in Health & Disease 2015; 26: 26382). US 2004/0170617A1 relates to a method of treating diseases associated with abnormal gastrointestinal flora. WO 2014/176632A1 relates to compositions and methods for treating microbiota-related psychotropic conditions and diseases.

### Summary of Invention

In alternative embodiments, provided are compositions, including formulations, pharmaceutical compositions, foods, feeds, supplements, products of manufacture, and the like, for use in treating, ameliorating, reversing and/or preventing (acting as a prophylaxis) autism, e.g., regressive autism, comprising a rixafimin and another antimicrobial or antibiotic agent, for example, vancomycin, metronidazole, tinidazole or a combination thereof.

In alternative embodiments, provided are compositions for use in treating, ameliorating, reversing and/or preventing (acting as a prophylaxis) an autism or an autism spectrum disorder (ASD), optionally a regressive autism setback-type autism, or an acquired autistic syndrome, in an individual in need thereof, the pharmaceutical preparation or the pharmaceutical composition comprising or consisting of:
a rifaximin (optionally a Xifaxan^{™}, XifaxanTA^{™} or NORMIX^{™}) and at least one additional antimicrobial or antibiotic agent,
whereinthe at least one additional antimicrobial or antibiotic agent comprises a vancomycin, a metronidazole (optionally FLAGYL^{™}, METRO^{™}), a tinidazole (optionally FASIGYN^{™}, SIMPLOTAN^{™}, TINDAMAX^{™}) or a combination thereof.

In alternative embodiments, the autism or the autism spectrum disorder (ASD) is selected from the group consisting of autistic disorder, pervasive developmental disorder not otherwise specified (PDD-NOS), and Asperger syndrome.

In alternative examples, the at least one additional antimicrobial or antibiotic agent comprises: an antibiotic or antibacterial agent from one or more of the following classes selected from: tetracyclines, penicillins, macrolides, quinolones, chloramphenicol, rifamycins, sulphonamides, co-trimoxazole, and oxazolidinones.

In alternative examples, the at least one additional antimicrobial or antibiotic agent comprises: a doxycycline, chlortetracycline, tetracycline hydrochloride, oxytetracycline, demeclocycline, methacycline, minocycline, penicillin, amoxycillin, erythromycin, clarithromycin, roxithromycin, azithromycin, spiramycin, oleandomycin, josamycin, kitsamysin, flurithromycin, nalidixic acid, oxolinic acid, norfloxacin, perfloxacin, amifloxacin, ofloxacin, ciprofloxacin, sparfloxacin, levofloxacin, rifabutin, rifampicin, rifapentin, sulfisoxazole, sulfamethoxazole, sulfadiazine, sulfadoxine, sulfasalazine, sulfaphenazole, dapsone, sulfacytidine, linezolid or any combination thereof.

In alternative examples, the at least one additional antimicrobial or antibiotic agent comprises:
an ampicillin, a sulbactama tetracycline, a cephalosporin, a carbapenem, an imipenem, a meropenem, a monobactam, a lincosamide, a clindamycin, a quinolone, a fluoroquinolone, a sulphonamide, a fradicin, a nitroimidazole, a metronidazole, a tinidazole, an anti-Clostridial agent, or a ramoplanan,
an aminoglycoside antibiotic, a gentamycin, a neomycin, a streptomycin, a paromomycin, a verdamicin, a mutamicin, a sisomicin, a netilmicin, a retymicin, a kanamycin, an amphenicol, an ansamycin, a beta-lactam (β-lactam) antibiotic, a carbapenem, a cephalosporin, a cephamycin, a monobactam, an oxacephem, a lincosamide antibiotic, a clindamycin, or a lincomycin,
a glycopeptide antibiotic, a vancomycin, a teicoplanin, a telavancin, a bleomycin, a ramoplanin, a decaplanin, a polypeptide antibiotic, an actinomycin, an actinomycin D, a bacitracin, a bacitracin, a tetracycline, a 2,4-diaminopyrimidine class antibiotic, a clavacin, a clairformin, a claviform, an expansine, a clavatin, an expansin, a gigantin, a leucopin, a patuline or a patulin), or an equivalent thereof or a combination thereof.

In alternative embodiments, the individual exhibits at least an about 5% to 10% reduction in autism or an autism spectrum disorder (ASD) symptom severity after administration of the formulation, pharmaceutical preparation or pharmaceutical composition to the individual in need thereof as compared to before initiating the administration, wherein the reduction in symptom severity is based on an assessment system selected from the group consisting of Childhood Autism Rating Scale (CARS), Childhood Autism Rating Scale 2--Standard Form (CARS2-ST), Childhood Autism Rating Scale 2--High Functioning (CARS2-HF), and a combination thereof.

In alternative embodiments, the at least about 5% to 10% reduction in autism or an autism spectrum disorder (ASD) symptom severity is achieved after about 1 to 2 or more weeks, or after about 1 to 2 months, of initiating the administration.

In alternative embodiments, the at least about 5% to 10% reduction in autism or an autism spectrum disorder (ASD) symptom severity is maintained for at least about 4 to 8 weeks after discontinuing the administration.

In alternative embodiments, the formulation, the pharmaceutical or the pharmaceutical preparation is formulated as a chewable delivery vehicle, a gum, a gummy, a candy, a lozenge, an ice cream or an ice, or a yogurt.

In alternative embodiments, a unit dosage is a pediatric unit dosage, and optionally the unit dosage is between about 10 mg and 1100 mgm, or is about 10, 20, 30, 40, 50, 60, 70, 75, 80, 90, 100, 125, 150, 175, 200, 225, 250, 275, 300, 325, 350, 375, 400, 425, 450, 475, 500, 600, 700, 750, 800, 900, 1000 or 1100 or more mg per unit dose.

In alternative embodiments, a daily dosage is about 100, 125, 150, 175, 200, 225, 250, 275, 300, 325, 350, 375, 400, 425, 450, 475, 500, 600, 700, 750, 800, 900, 1000 or 1100 or more mg per day, or between about 100 and 1100 mgm per day.

In alternative embodiments, a unit dosage is set for bid (twice a day), tid (three times a day), four times a day, five times a day or six times a day or more, with the unit dosage and daily dosage adjusted to be: about 1000 mg/70 kg a day, or about 14 mg/kg a day, for an adult median dose per day; or for a pediatric dosage about 350 mg/25 kg a day, or about 15 to 16 mg/kg, a day; or equivalent.

In alternative embodiments, the formulation, the pharmaceutical or the pharmaceutical preparation further comprises a flavoring or a sweetening agent, an aspartame, a stevia, monk fruit, a sucralose, a saccharin, a cyclamate, a xylitol, a vanilla, an artificial vanilla or chocolate or strawberry flavor, an artificial chocolate essence, or a mixture or combination thereof.

In alternative embodiments, the formulation, the pharmaceutical or the pharmaceutical preparation further comprises a preservative, a benzoic acid or a potassium sorbate.

In alternative embodiments, the formulation, the pharmaceutical or the pharmaceutical preparation further comprises, or has added to: at least one probiotic or prebiotic, wherein optionally the prebiotic comprises an inulin, lactulose, extracts of artichoke, chicory root, oats, barley, various legumes, garlic, kale, beans or flax or an herb, wherein optionally the probiotic comprises a cultured or stool-extracted microorganism or bacteria, or a bacterial component, and optionally the bacteria or bacterial component comprises or is derived from a *Bacteroidetes, a Firmicutes, a Lactobacilli, a Bifidobacteria,* an *E coli, a Strep fecalis* and equivalents.

In alternative embodiments, the formulation, the pharmaceutical or the pharmaceutical preparation further comprises, or has added to: at least one congealing agent, wherein optionally the congealing agent comprises an arrowroot or a plant starch, a powdered flour, a powdered potato or potato starch, an absorbant polymer, an Absorbable Modified Polymer, and/or a corn flour or a corn starch.

In alternative embodiments, the formulation, the pharmaceutical or the pharmaceutical preparation further comprises an additive selected from one or more of a saline, a media, a defoaming agent, a surfactant agent, a lubricant, an acid neutralizer, a marker, a cell marker, a drug, an antibiotic, a contrast agent, a dispersal agent, a buffer or a buffering agent, a sweetening agent, a debittering agent, a flavoring agent, a pH stabilizer, an acidifying agent, a preservative, a desweetening agent and/or coloring agent, vitamin, mineral and/or dietary supplement, or a prebiotic nutrient.

In alternative embodiments, the formulation, the pharmaceutical or the pharmaceutical preparation further comprises, or has added to: at least one Biofilm Disrupting Compound, wherein optionally the biofilm disrupting compound comprises an enzyme, a deoxyribonuclease (DNase), N-acetylcysteine, an auranofin, an alginate lyase, glycoside hydrolase dispersin B; a Quorum-sensing inhibitor, a ribonucleic acid III inhibiting peptide, *Salvadora persica* extracts, Competence-stimulating peptide, Patulin and penicillic acid; peptides - cathelicidin-derived peptides, small lytic peptide, PTP-7, Nitric oxide, neo-emulsions; ozone, lytic bacteriophages, lactoferrin, xylitol hydrogel, synthetic iron chelators, cranberry components, curcumin, silver nanoparticles, Acetyl-11-keto-β-boswellic acid (AKBA), barley coffee components, probiotics, sinefungin, S-adenosylmethionine, S-adenosyl-homocysteine, *Delisea* furanones, N-sulfonyl homoserine lactones or any combination thereof.

In alternative embodiments, the formulation, the pharmaceutical or the pharmaceutical preparation is formulated as a delayed or gradual enteric release composition or formulation, and optionally the formulation comprises a gastro-resistant coating designed to dissolve at a pH of 7 in the terminal ileum, e.g., an active ingredient is coated with an acrylic based resin or equivalent, e.g., a poly(meth)acrylate, e.g. a methacrylic acid copolymer B, NF, which dissolves at pH 7 or greater, e.g., comprises a multimatrix (MMX) formulation.

In alternative examples, the formulation, the pharmaceutical or the pharmaceutical preparation is contained in a delivery vehicle, product of manufacture, container, syringe, device or bag.

In alternative embodiments, the formulation, the pharmaceutical or the pharmaceutical preparation is initially manufactured or formulated as a liquid, a suspension, a gel, a geltab, a semisolid, a tablet, a sachet, a lozenge or a capsule, or as an enteral formulation, or re-formulated for final delivery as a liquid, a suspension, a gel, a geltab, a semisolid, a tablet, a sachet, a lozenge or a capsule, or as an enteral formulation.

In a first example, forms described herein and not according to the claims include the following:
A method for treating, ameliorating, reversing and/or preventing (acting as a prophylaxis) an autism or an autism spectrum disorder (ASD), optionally a regressive autism setback-type autism, or an acquired autistic syndrome, in an individual in need thereof, comprising administering to an individual in need thereof a formulation, a pharmaceutical preparation or a pharmaceutical composition comprising or consisting of:
(a) a rifaximin (optionally a Xifaxan^{™}, XifaxanTA^{™} or NORMIX^{™}), an extended intestinal release (EIR) rifaximin, a rifamycin derivative, a rifampicin (or rifampin) (optionally RIFADIN^{™}), a rifabutin (optionally MYCOBUTIN^{™}), a rifapentine (optionally Priftin^{™}), a rifalazil, a bicozamycin, or a mixture or combination thereof, or
(b) a rifaximin (optionally a Xifaxan^{™}, XifaxanTA^{™} or NORMIX^{™}) and at least one additional antimicrobial or antibiotic agent,
wherein optionally the at least one additional antimicrobial or antibiotic agent comprises a vancomycin, a metronidazole (optionally Flagyl^{™}, Metro^{™}), a tinidazole (optionally Fasigyn^{™}, Simplotan^{™}, Tindamax^{™}) or a combination thereof.

In an example of the above method, the autism or the autism spectrum disorder (ASD) is selected from the group consisting of autistic disorder, pervasive developmental disorder not otherwise specified (PDD-NOS), and Asperger syndrome.

In one or more examples of the above method, the at least one additional antimicrobial or antibiotic agent comprises: an antibiotic or antibacterial agent from one or more of the following classes selected from: tetracyclines, penicillins, macrolides, quinolones, chloramphenicol, rifamycins, sulphonamides, co-trimoxazole, and oxazolidinones.

In one or more examples of the above method, the at least one additional antimicrobial or antibiotic agent comprises: a doxycycline, chlortetracycline, tetracycline hydrochloride, oxytetracycline, demeclocycline, methacycline, minocycline, penicillin, amoxycillin, erythromycin, clarithromycin, roxithromycin, azithromycin, spiramycin, oleandomycin, josamycin, kitsamysin, flurithromycin, nalidixic acid, oxolinic acid, norfloxacin, perfloxacin, amifloxacin, ofloxacin, ciprofloxacin, sparfloxacin, levofloxacin, rifabutin, rifampicin, rifapentin, sulfisoxazole, sulfamethoxazole, sulfadiazine, sulfadoxine, sulfasalazine, sulfaphenazole, dapsone, sulfacytidine, linezolid or any combination thereof.

In one or more examples of the above method, the at least one additional antimicrobial or antibiotic agent comprises:
(a) an ampicillin, a sulbactama tetracycline, a cephalosporin, a carbapenem, an imipenem, a meropenem, a monobactam, a lincosamide, a clindamycin, a quinolone, a fluoroquinolone, a sulphonamide, a fradicin, a nitroimidazole, a metronidazole, a tinidazole, an anti-Clostridial agent, or a ramoplanan,
(b) an minoglycoside antibiotic, a gentamycin, a neomycin, a streptomycin, a paromomycin, a verdamicin, a mutamicin, a sisomicin, a netilmicin, a retymicin, a kanamycin, an amphenicol, an ansamycin, a beta-lactam (β-lactam) antibiotic, a carbapenem, a cephalosporin, a cephamycin, a monobactam, an oxacephem, a lincosamide antibiotic, a clindamycin, or a lincomycin,
(c) a lycopeptide antibiotic, a vancomycin, a teicoplanin, a telavancin, a bleomycin, a ramoplanin, a decaplanin, a polypeptide antibiotic, an actinomycin, an actinomycin D, a bacitracin, a bacitracin, a tetracycline, a 2,4-diaminopyrimidine class antibiotic, a clavacin, a clairformin, a claviform, an expansine, a clavatin, an expansin, a gigantin, a leucopin, a patuline or a patulin), or
(d) an quivalent thereof or a combination thereof.

In one or more examples of the above method, the individual exhibits at least an about 5% to 10% reduction in autism or an autism spectrum disorder (ASD) symptom severity after administration of the formulation, pharmaceutical preparation or pharmaceutical composition to the individual in need thereof as compared to before initiating the administration, wherein the reduction in symptom severity is based on an assessment system selected from the group consisting of Childhood Autism Rating Scale (CARS), Childhood Autism Rating Scale 2-Standard Form (CARS2-ST), Childhood Autism Rating Scale 2--High Functioning (CARS2-HF), and a combination thereof.

In one or more examples of the above method, the formulation, the pharmaceutical or the pharmaceutical preparation is formulated as a chewable delivery vehicle, a gum, a gummy, a candy, a lozenge, an ice cream or an ice, or a yogurt.

In one or more examples of the above method, a unit dosage is a pediatric unit dosage, and optionally the unit dosage is between about 10 mg and 1100 mgm, or is about 10, 20, 30, 40, 50, 60, 70, 75, 80, 90, 100, 125, 150, 175, 200, 225, 250, 275, 300, 325, 350, 375, 400, 425, 450, 475, 500, 600, 700, 750, 800, 900, 1000 or 1100 or more mg per unit dose.

In one or more examples of the above method, a daily dosage is about 100, 125, 150, 175, 200, 225, 250, 275, 300, 325, 350, 375, 400, 425, 450, 475, 500, 600, 700, 750, 800, 900, 1000 or 1100 or more mg per day, or between about 100 and 1100 mgm per day.

In one or more examples of the above method, a unit dosage is set for bid (twice a day), tid (three times a day), four times a day, five times a day or six times a day or more, with the unit dosage and daily dosage adjusted to be: about 1000 mg/70 kg a day, or about 14 mg/kg a day, for an adult median dose per day; or for a pediatric dosage about 350 mg/25 kg a day, or about 15 to 16 mg/kg, a day; or equivalent.

In one or more examples of the above method, the formulation, the pharmaceutical or the pharmaceutical preparation further comprises a flavoring or a sweetening agent, an aspartame, a stevia, monk fruit, a sucralose, a saccharin, a cyclamate, a xylitol, a vanilla, an artificial vanilla or chocolate or strawberry flavor, an artificial chocolate essence, or a mixture or combination thereof.

In one or more examples of the above method, the formulation, the pharmaceutical or the pharmaceutical preparation further comprises a preservative, a benzoic acid or a potassium sorbate.

In one or more examples of the above method, the formulation, the pharmaceutical or the pharmaceutical preparation further comprises, or has added to: at least one probiotic or prebiotic, wherein optionally the prebiotic comprises an inulin, lactulose, extracts of artichoke, chicory root, oats, barley, various legumes, garlic, kale, beans or flax or an herb, wherein optionally the probiotic comprises a cultured or stool-extracted microorganism or bacteria, or a bacterial component, and optionally the bacteria or bacterial component comprises or is derived from a *Bacteroidetes,* a *Firmicutes,* a *Lactobacilli,* a *Bifidobacteria,* an *E coli,* a *Strep fecalis* and equivalents.

In one or more examples of the above method, the formulation, the pharmaceutical or the pharmaceutical preparation further comprises, or has added to: at least one congealing agent, wherein optionally the congealing agent comprises an arrowroot or a plant starch, a powdered flour, a powdered potato or potato starch, an absorbant polymer, an Absorbable Modified Polymer, and/or a corn flour or a corn starch.

In one or more examples of the above method, the formulation, the pharmaceutical or the pharmaceutical preparation further comprises an additive selected from one or more of a saline, a media, a defoaming agent, a surfactant agent, a lubricant, an acid neutralizer, a marker, a cell marker, a drug, an antibiotic, a contrast agent, a dispersal agent, a buffer or a buffering agent, a sweetening agent, a debittering agent, a flavoring agent, a pH stabilizer, an acidifying agent, a preservative, a desweetening agent and/or coloring agent, vitamin, mineral and/or dietary supplement, or a prebiotic nutrient.

In one or more examples of the above method, the formulation, the pharmaceutical or the pharmaceutical preparation further comprises, or has added to: at least one Biofilm Disrupting Compound, wherein optionally the biofilm disrupting compound comprises an enzyme, a deoxyribonuclease (DNase), N-acetylcysteine, an auranofin, an alginate lyase, glycoside hydrolase dispersin B; a Quorum-sensing inhibitor, a ribonucleic acid III inhibiting peptide, *Salvadora persica* extracts, Competence-stimulating peptide, Patulin and penicillic acid; peptides - cathelicidin-derived peptides, small lytic peptide, PTP-7, Nitric oxide, neo-emulsions; ozone, lytic bacteriophages, lactoferrin, xylitol hydrogel, synthetic iron chelators, cranberry components, curcumin, silver nanoparticles, Acetyl-11-keto-β-boswellic acid (AKBA), barley coffee components, probiotics, sinefungin, S-adenosylmethionine, S-adenosyl-homocysteine, *Delisea* furanones, N-sulfonyl homoserine lactones or any combination thereof.

In one or more examples of the above method, the formulation, the pharmaceutical or the pharmaceutical preparation is formulated as a delayed or gradual enteric release composition or formulation, and optionally the formulation comprises a gastro-resistant coating designed to dissolve at a pH of 7 in the terminal ileum, e.g., an active ingredient is coated with an acrylic based resin or equivalent, e.g., a poly(meth)acrylate, e.g. a methacrylic acid copolymer B, NF, which dissolves at pH 7 or greater, e.g., comprises a multimatrix (MMX) formulation.

In one or more examples of the above method, the formulation, the pharmaceutical or the pharmaceutical preparation is contained in a delivery vehicle, product of manufacture, container, syringe, device or bag.

In one or more examples of the above method, the formulation, the pharmaceutical or the pharmaceutical preparation is initially manufactured or formulated as a liquid, a suspension, a gel, a geltab, a semisolid, a tablet, a sachet, a lozenge or a capsule, or as an enteral formulation, or re-formulated for final delivery as a liquid, a suspension, a gel, a geltab, a semisolid, a tablet, a sachet, a lozenge or a capsule, or as an enteral formulation.

In a second example, forms described herein and not according to the claims include the following:
Use of
(a) a rifaximin (optionally a Xifaxan^{™}, XifaxanTA^{™} or NORMIX^{™}), an extended intestinal release (EIR) rifaximin, a rifamycin derivative, a rifampicin (or rifampin) (optionally RIFADIN^{™}), a rifabutin (optionally MYCOBUTIN^{™}), a rifapentine (optionally Priftin^{™}), a rifalazil, a bicozamycin, or a mixture or combination thereof, or
(b) a rifaximin (optionally a Xifaxan^{™}, XifaxanTA^{™} or NORMIX^{™}) and at least one additional antimicrobial or antibiotic agent,

wherein optionally the at least one additional antimicrobial or antibiotic agent comprises a vancomycin, a metronidazole (optionally Flagyl^{™}, Metro^{™}), a tinidazole (optionally Fasigyn^{™}, Simplotan^{™}, Tindamax^{™}) or a combination thereof,
in the manufacture of a medicament for treating, ameliorating, reversing and/or preventing (acting as a prophylaxis) an autism or an autism spectrum disorder (ASD), optionally a regressive autism setback-type autism, or an acquired autistic syndrome, in an individual in need thereof.

In an example of the use above, the autism or the autism spectrum disorder (ASD) is selected from the group consisting of autistic disorder, pervasive developmental disorder not otherwise specified (PDD-NOS), and Asperger syndrome.

In one or more examples of the above use, the at least one additional antimicrobial or antibiotic agent comprises: an antibiotic or antibacterial agent from one or more of the following classes selected from: tetracyclines, penicillins, macrolides, quinolones, chloramphenicol, rifamycins, sulphonamides, co-trimoxazole, and oxazolidinones.

In one or more examples of the above use, the at least one additional antimicrobial or antibiotic agent comprises: a doxycycline, chlortetracycline, tetracycline hydrochloride, oxytetracycline, demeclocycline, methacycline, minocycline, penicillin, amoxycillin, erythromycin, clarithromycin, roxithromycin, azithromycin, spiramycin, oleandomycin, josamycin, kitsamysin, flurithromycin, nalidixic acid, oxolinic acid, norfloxacin, perfloxacin, amifloxacin, ofloxacin, ciprofloxacin, sparfloxacin, levofloxacin, rifabutin, rifampicin, rifapentin, sulfisoxazole, sulfamethoxazole, sulfadiazine, sulfadoxine, sulfasalazine, sulfaphenazole, dapsone, sulfacytidine, linezolid or any combination thereof.

In one or more examples of the above use, the at least one additional antimicrobial or antibiotic agent comprises:
(a) an ampicillin, a sulbactama tetracycline, a cephalosporin, a carbapenem, an imipenem, a meropenem, a monobactam, a lincosamide, a clindamycin, a quinolone, a fluoroquinolone, a sulphonamide, a fradicin, a nitroimidazole, a metronidazole, a tinidazole, an anti-Clostridial agent, or a ramoplanan,
(b) an aminoglycoside antibiotic, a gentamycin, a neomycin, a streptomycin, a paromomycin, a verdamicin, a mutamicin, a sisomicin, a netilmicin, a retymicin, a kanamycin, an amphenicol, an ansamycin, a beta-lactam (β-lactam) antibiotic, a carbapenem, a cephalosporin, a cephamycin, a monobactam, an oxacephem, a lincosamide antibiotic, a clindamycin, or a lincomycin,
(c) a glycopeptide antibiotic, a vancomycin, a teicoplanin, a telavancin, a bleomycin, a ramoplanin, a decaplanin, a polypeptide antibiotic, an actinomycin, an actinomycin D, a bacitracin, a bacitracin, a tetracycline, a 2,4-diaminopyrimidine class antibiotic, a clavacin, a clairformin, a claviform, an expansine, a clavatin, an expansin, a gigantin, a leucopin, a patuline or a patulin), or
(d) an equivalent thereof or a combination thereof.

In one or more examples of the above use, the individual exhibits at least an about 5% to 10% reduction in autism or an autism spectrum disorder (ASD) symptom severity after administration of the medicament to the individual in need thereof as compared to before initiating the administration, wherein the reduction in symptom severity is based on an assessment system selected from the group consisting of Childhood Autism Rating Scale (CARS), Childhood Autism Rating Scale 2--Standard Form (CARS2-ST), Childhood Autism Rating Scale 2--High Functioning (CARS2-HF), and a combination thereof.

In one or more examples of the above use, the medicament is formulated as a chewable delivery vehicle, a gum, a gummy, a candy, a lozenge, an ice cream or an ice, or a yogurt.

In one or more examples of the above use, the medicament is formulated as a pediatric unit dosage, and optionally the unit dosage is between about 10 mg and 1100 mgm, or is about 10, 20, 30, 40, 50, 60, 70, 75, 80, 90, 100, 125, 150, 175, 200, 225, 250, 275, 300, 325, 350, 375, 400, 425, 450, 475, 500, 600, 700, 750, 800, 900, 1000 or 1100 or more mg per unit dose.

In one or more examples of the above use, the medicament is formulated for a daily dosage of about 100, 125, 150, 175, 200, 225, 250, 275, 300, 325, 350, 375, 400, 425, 450, 475, 500, 600, 700, 750, 800, 900, 1000 or 1100 or more mg per day, or between about 100 and 1100 mgm per day.

In one or more examples of the above use, the medicament is formulated for administration bid (twice a day), tid (three times a day), four times a day, five times a day or six times a day or more, with the unit dosage and daily dosage adjusted to be: about 1000 mg/70 kg a day, or about 14 mg/kg a day, for an adult median dose per day; or for a pediatric dosage about 350 mg/25 kg a day, or about 15 to 16 mg/kg, a day; or equivalent.

In one or more examples of the above use, the medicament further comprises a flavoring or a sweetening agent, an aspartame, a stevia, monk fruit, a sucralose, a saccharin, a cyclamate, a xylitol, a vanilla, an artificial vanilla or chocolate or strawberry flavor, an artificial chocolate essence, or a mixture or combination thereof.

In one or more examples of the above use, the medicament further comprises a preservative, a benzoic acid or a potassium sorbate.

In one or more examples of the above use, the medicament further comprises, or has added to: at least one probiotic or prebiotic, wherein optionally the prebiotic comprises an inulin, lactulose, extracts of artichoke, chicory root, oats, barley, various legumes, garlic, kale, beans or flax or an herb, wherein optionally the probiotic comprises a cultured or stool-extracted microorganism or bacteria, or a bacterial component, and optionally the bacteria or bacterial component comprises or is derived from a *Bacteroidetes,* a *Firmicutes,* a *Lactobacilli,* a *Bifidobacteria,* an *E coli*, a *Strep fecalis* and equivalents.

In one or more examples of the above use, the medicament further comprises, or has added to: at least one congealing agent, wherein optionally the congealing agent comprises an arrowroot or a plant starch, a powdered flour, a powdered potato or potato starch, an absorbant polymer, an Absorbable Modified Polymer, and/or a corn flour or a corn starch.

In one or more examples of the above use, the medicament further comprises an additive selected from one or more of a saline, a media, a defoaming agent, a surfactant agent, a lubricant, an acid neutralizer, a marker, a cell marker, a drug, an antibiotic, a contrast agent, a dispersal agent, a buffer or a buffering agent, a sweetening agent, a debittering agent, a flavoring agent, a pH stabilizer, an acidifying agent, a preservative, a desweetening agent and/or coloring agent, vitamin, mineral and/or dietary supplement, or a prebiotic nutrient.

In one or more examples of the above use, the medicament further comprises, or has added to: at least one Biofilm Disrupting Compound, wherein optionally the biofilm disrupting compound comprises an enzyme, a deoxyribonuclease (DNase), N-acetylcysteine, an auranofin, an alginate lyase, glycoside hydrolase dispersin B; a Quorum-sensing inhibitor, a ribonucleic acid III inhibiting peptide, *Salvadora persica* extracts, Competence-stimulating peptide, Patulin and penicillic acid; peptides - cathelicidin-derived peptides, small lytic peptide, PTP-7, Nitric oxide, neo-emulsions; ozone, lytic bacteriophages, lactoferrin, xylitol hydrogel, synthetic iron chelators, cranberry components, curcumin, silver nanoparticles, Acetyl-11-keto-β-boswellic acid (AKBA), barley coffee components, probiotics, sinefungin, S-adenosylmethionine, S-adenosyl-homocysteine, *Delisea* furanones, N-sulfonyl homoserine lactones or any combination thereof.

In one or more examples of the above use, the medicament is formulated as a delayed or gradual enteric release composition or formulation, and optionally the formulation comprises a gastro-resistant coating designed to dissolve at a pH of 7 in the terminal ileum, e.g., an active ingredient is coated with an acrylic based resin or equivalent, e.g., a poly(meth)acrylate, e.g. a methacrylic acid copolymer B, NF, which dissolves at pH 7 or greater, e.g., comprises a multimatrix (MMX) formulation.

In one or more examples of the above use, the medicament is contained in a delivery vehicle, product of manufacture, container, syringe, device or bag.

In one or more examples of the above use, the medicament is initially manufactured or formulated as a liquid, a suspension, a gel, a geltab, a semisolid, a tablet, a sachet, a lozenge or a capsule, or as an enteral formulation, or re-formulated for final delivery as a liquid, a suspension, a gel, a geltab, a semisolid, a tablet, a sachet, a lozenge or a capsule, or as an enteral formulation.

In an aspect, forms of the invention described herein include the following:
A composition comprising or consisting of:
a rifaximin (optionally a Xifaxan^{™}, XifaxanTA^{™} or NORMIX^{™}) and at least one additional antimicrobial or antibiotic agent,
wherein the at least one additional antimicrobial or antibiotic agent comprises a vancomycin, a metronidazole (optionally Flagyl^{™}, Metro^{™}), a tinidazole (optionally Fasigyn^{™}, Simplotan^{™}, Tindamax^{™}) or a combination thereof,
for use in treating, or preventing (acting as a prophylaxis) an autism or an autism spectrum disorder (ASD), optionally a regressive autism setback-type autism, or an acquired autistic syndrome, in an individual in need thereof.

In one or more examples of the above composition, the autism or the autism spectrum disorder (ASD) is selected from the group consisting of autistic disorder, pervasive developmental disorder not otherwise specified (PDD-NOS), and Asperger syndrome.

In one or more examples of the above composition, the at least one additional antimicrobial or antibiotic agent comprises: an antibiotic or antibacterial agent from one or more of the following classes selected from: tetracyclines, penicillins, macrolides, quinolones, chloramphenicol, rifamycins, sulphonamides, co-trimoxazole, and oxazolidinones.

In one or more examples of the above composition, the at least one additional antimicrobial or antibiotic agent comprises: a doxycycline, chlortetracycline, tetracycline hydrochloride, oxytetracycline, demeclocycline, methacycline, minocycline, penicillin, amoxycillin, erythromycin, clarithromycin, roxithromycin, azithromycin, spiramycin, oleandomycin, josamycin, kitsamysin, flurithromycin, nalidixic acid, oxolinic acid, norfloxacin, perfloxacin, amifloxacin, ofloxacin, ciprofloxacin, sparfloxacin, levofloxacin, rifabutin, rifampicin, rifapentin, sulfisoxazole, sulfamethoxazole, sulfadiazine, sulfadoxine, sulfasalazine, sulfaphenazole, dapsone, sulfacytidine, linezolid or any combination thereof.

In one or more examples of the above composition the at least one additional antimicrobial or antibiotic agent comprises:
(a) an ampicillin, a sulbactama tetracycline, a cephalosporin, a carbapenem, an imipenem, a meropenem, a monobactam, a lincosamide, a clindamycin, a quinolone, a fluoroquinolone, a sulphonamide, a fradicin, a nitroimidazole, a metronidazole, a tinidazole, an anti-Clostridial agent, or a ramoplanan,
(b) an aminoglycoside antibiotic, a gentamycin, a neomycin, a streptomycin, a paromomycin, a verdamicin, a mutamicin, a sisomicin, a netilmicin, a retymicin, a kanamycin, an amphenicol, an ansamycin, a beta-lactam (β-lactam) antibiotic, a carbapenem, a cephalosporin, a cephamycin, a monobactam, an oxacephem, a lincosamide antibiotic, a clindamycin, or a lincomycin,
(c) a glycopeptide antibiotic, a vancomycin, a teicoplanin, a telavancin, a bleomycin, a ramoplanin, a decaplanin, a polypeptide antibiotic, an actinomycin, an actinomycin D, a bacitracin, a bacitracin, a tetracycline, a 2,4-diaminopyrimidine class antibiotic, a clavacin, a clairformin, a claviform, an expansine, a clavatin, an expansin, a gigantin, a leucopin, a patuline or a patulin), or
(d) an equivalent thereof or a combination thereof.

In one or more examples of the above composition, the individual exhibits at least an about 5% to 10% reduction in autism or an autism spectrum disorder (ASD) symptom severity after administration of the composition to the individual in need thereof as compared to before initiating the administration, wherein the reduction in symptom severity is based on an assessment system selected from the group consisting of Childhood Autism Rating Scale (CARS), Childhood Autism Rating Scale 2--Standard Form (CARS2-ST), Childhood Autism Rating Scale 2--High Functioning (CARS2-HF), and a combination thereof.

In one or more examples of the above composition, the composition is formulated as a chewable delivery vehicle, a gum, a gummy, a candy, a lozenge, an ice cream or an ice, or a yogurt.

In one or more examples of the above composition, a unit dosage of the composition is a pediatric unit dosage, and optionally the unit dosage is between about 10 mg and 1100 mgm, or is about 10, 20, 30, 40, 50, 60, 70, 75, 80, 90, 100, 125, 150, 175, 200, 225, 250, 275, 300, 325, 350, 375, 400, 425, 450, 475, 500, 600, 700, 750, 800, 900, 1000 or 1100 or more mg per unit dose.

In one or more examples of the above composition, a daily dosage of the composition is about 100, 125, 150, 175, 200, 225, 250, 275, 300, 325, 350, 375, 400, 425, 450, 475, 500, 600, 700, 750, 800, 900, 1000 or 1100 or more mg per day, or between about 100 and 1100 mgm per day.

In one or more examples of the above composition, a unit dosage of the composition is set for bid (twice a day), tid (three times a day), four times a day, five times a day or six times a day or more, with the unit dosage and daily dosage adjusted to be: about 1000 mg/70 kg a day, or about 14 mg/kg a day, for an adult median dose per day; or for a pediatric dosage about 350 mg/25 kg a day, or about 15 to 16 mg/kg, a day; or equivalent.

In one or more examples, the composition further comprising a flavoring or a sweetening agent, an aspartame, a stevia, monk fruit, a sucralose, a saccharin, a cyclamate, a xylitol, a vanilla, an artificial vanilla or chocolate or strawberry flavor, an artificial chocolate essence, or a mixture or combination thereof.

In one or more examples, the composition further comprising a preservative, a benzoic acid or a potassium sorbate.

In one or more examples, the composition further comprising: at least one probiotic or prebiotic, wherein optionally the prebiotic comprises an inulin, lactulose, extracts of artichoke, chicory root, oats, barley, various legumes, garlic, kale, beans or flax or an herb, wherein optionally the probiotic comprises a cultured or stool-extracted microorganism or bacteria, or a bacterial component, and optionally the bacteria or bacterial component comprises or is derived from a *Bacteroidetes*, a *Firmicutes, a Lactobacilli,* a *Bifidobacteria,* an *E coli,* a *Strep fecalis* and equivalents.

In one or more examples, the composition further comprising: at least one congealing agent, wherein optionally the congealing agent comprises an arrowroot or a plant starch, a powdered flour, a powdered potato or potato starch, an absorbant polymer, an Absorbable Modified Polymer, and/or a corn flour or a corn starch.

In one or more examples, the composition further comprising an additive selected from one or more of a saline, a media, a defoaming agent, a surfactant agent, a lubricant, an acid neutralizer, a marker, a cell marker, a drug, an antibiotic, a contrast agent, a dispersal agent, a buffer or a buffering agent, a sweetening agent, a debittering agent, a flavoring agent, a pH stabilizer, an acidifying agent, a preservative, a desweetening agent and/or coloring agent, vitamin, mineral and/or dietary supplement, or a prebiotic nutrient.

In one or more examples, the above composition further comprising: at least one Biofilm Disrupting Compound, wherein optionally the biofilm disrupting compound comprises an enzyme, a deoxyribonuclease (DNase), N-acetylcysteine, an auranofin, an alginate lyase, glycoside hydrolase dispersin B; a Quorum-sensing inhibitor, a ribonucleic acid III inhibiting peptide, *Salvadora persica* extracts, Competence-stimulating peptide, Patulin and penicillic acid; peptides - cathelicidin-derived peptides, small lytic peptide, PTP-7, Nitric oxide, neo-emulsions; ozone, lytic bacteriophages, lactoferrin, xylitol hydrogel, synthetic iron chelators, cranberry components, curcumin, silver nanoparticles, Acetyl-11-keto-β-boswellic acid (AKBA), barley coffee components, probiotics, sinefungin, S-adenosylmethionine, S-adenosyl-homocysteine, *Delisea* furanones, N-sulfonyl homoserine lactones or any combination thereof.

In one or more examples of the above composition, the composition is formulated as a delayed or gradual enteric release composition or formulation, and optionally the formulation comprises a gastro-resistant coating designed to dissolve at a pH of 7 in the terminal ileum, e.g., an active ingredient is coated with an acrylic based resin or equivalent, e.g., a poly(meth)acrylate, e.g. a methacrylic acid copolymer B, NF, which dissolves at pH 7 or greater, e.g., comprises a multimatrix (MMX) formulation.

In one or more examples of the above composition, the composition is contained in a delivery vehicle, product of manufacture, container, syringe, device or bag.

In one or more examples of the above composition, the composition is initially manufactured or formulated as a liquid, a suspension, a gel, a geltab, a semisolid, a tablet, a sachet, a lozenge or a capsule, or as an enteral formulation, or re-formulated for final delivery as a liquid, a suspension, a gel, a geltab, a semisolid, a tablet, a sachet, a lozenge or a capsule, or as an enteral formulation.

The details of one or more embodiments of the invention are set forth in the accompanying description below. Other features, objects, and advantages of the invention will be apparent from the description and the claims.

### Description of Embodiments

In alternative embodiments, provided are pharmaceutical compositions for use in treating or preventing (acting as a prophylaxis) autism or an autism spectrum disorder (ASD), e.g., regressive autism. In alternative examples, these pharmaceutical compositions are dosaged and administered to children in need thereof. In alternative examples, pharmaceutical compositions are dosaged, formulated and dosaged as solid, liquid or aerosol preparations or formulations. In alternative embodiments, pharmaceutical compositions comprise rixafimin and other antimicrobial or antibiotic agent, for example, vancomycin, metronidazole, tinidazole or a combination thereof.

In alternative examples, antibiotics and antibacterials used to practice methods as provided herein are formulated and dosaged for oral administration as a powder, e.g., a lyophilised powder, which can be inserted into carriers, e.g., capsules, tablets, geltabs, and the like, e.g., for administration to autistic infants or children (or those suspected of developing autism) to ingest.

Because autism may present itself at the age of 2.5 years or above the children are unlikely to be able to swallow a capsule; thus, this provided are additional delivery vehicles, products of manufacture and devices to be combined with formulations as provided herein, e.g., powders such as lyophilised powders, e.g., lyophilised powder in a storage vehicle, e.g., capsules, geltabs and the like; for example, provided are delivery vehicles, products of manufacture and devices manufactured as a container, a kit, a package or a pack of a "device and capsule" together, e.g., operably associated such that the container, kit, package or a pack permits individuals, e.g., the very young children and the older children (and including disabled or handicapped individuals) to ingest the product, e.g., the lyophilised product, from the storage vehicle, e.g., capsules, geltabs and the like.

In alternative examples, the container, kit, a package or a pack provides the ability of any age child (or disabled or handicapped individual) to ingest or swallow the product within the storage vehicle (e.g., capsule) by "draining", e.g., by puncturing, crushing or otherwise opening, the storage vehicle using a puncturing, crushing or equivalent device (operably built into the container, kit, package or pack), and allowing passage or contact of the contents of the storage vehicle to an ingestible liquid, which is also contained within the container, kit, package or pack, which can be initially (before the puncturing, crushing or otherwise opening) in a separate compartment from the storage compartment. This puncturing, crushing or otherwise opening of the storage compartment and the passage or contact of the contents of the storage vehicle to the ingestible liquid effectively places the contents of the storage, e.g., a powder, into the ingestible liquid, which can be e.g., water, a milk, a yoghurt, an ice cream, a yogurt, a juice (e.g., a fruit juice, an apple juice) or a masking drink. The container, kit, package or pack can be designed as an infant feeding bottle, e.g., comprising a nipple or teat for the very young.

In alternative examples, this simple puncturing or crushing device allows the storage containers, e.g., geltabs or capsules, to be punctured and/or crushed or otherwise "opened", allowing the contents of the storage container, e.g., the powder, to fall out in to the liquid compartment, e.g., to the bottom end of a device or straight into a bottle or a container held underneath or configured to be attached and underneath. For example, in this way a provider, e.g., the mother, can purchase a supply of storage containers, e.g., geltabs or capsules, convert them as needed into a powder capable of being mixed a liquid of her choice that the child will be ingesting.

**In** alternative examples, for those capable of swallowing tablets, capsules and the like, the storage containers, e.g., geltabs, tablets or capsules, are manufactured as enteric coated to bypass the acid of the stomach and bile of the duodenum, such that the storage containers, e.g., geltabs, tablets or capsules open (e.g., dissolve) in the jejunum or below.

In alternative examples, further provided are instructions for use, e.g., that when emptied into a drink, providers (e.g., the mothers of infants or children) are advised to choose a drink that has its own buffering capacity such as flavoured milk, chocolate milk, ice cream, yoghurt, ice blocks, frozen icicles, or simply milk, e.g., that is being fed to the infant or child by a bottle, e.g., a milk bottle, with a nipple or teat.

In alternative examples, storage containers, e.g., geltabs, tablets or capsules, or any formulation as provided herein, also comprises an antacid, e.g., a calcium carbonate, magnesium hydroxide, propylene glycol alginate and sodium alginate, or the combination of aluminium hydroxide with magnesium trisilicate, magnesium oxide or magnesium carbonate, so that when the storage container is punctured, crushed or otherwise opened and put into contact with the liquid, e.g., the feeding bottle, and ingested, there will be greater protection from acid damage. In alternative examples, methods and instructions further comprise the infant or child also being given an acid suppressant beforehand to permit more viable living bacteria to arrive in the colon.

In alternative examples, formulations, pharmaceuticals or pharmaceutical preparations as provided herein are formulated or manufactured as storage vehicles, e.g., tablets, geltabs, pills, capsules and the like; and in alternative examples, these storage vehicles are contained in, or contained in a kit with, or packaged with, or sold together with, a storage vehicle 'cracking', puncturing, or otherwise opening or releasing device (, e.g., as a powder, e.g., as lyophilised material). These can be dispensed together, or configured together, or manufactured together, as a simple way of meeting the needs of both infants, the very young, older children and needful (e.g., handicapped) adults; e.g., as a powder, e.g., as lyophilised material, e.g., from their storage vehicles, e.g., as encapsulated formulations, pharmaceuticals or pharmaceutical preparations, thus permitting successful clinical administration on a frequent, e.g., bid, tid, or daily, basis for prolonged periods.

### Methods of use and applications of devices and compositions

In alternative examples, provided are compositions, devices and methods for treating, ameliorating, reversing and/or preventing (acting as a prophylaxis) autisms, including the so-called regressive autism, autism with regression, autistic regression, setback-type autism, or acquired autistic syndrome. In alternative examples, provided are compositions, devices and methods for treating, ameliorating, reversing and/or preventing (acting as a prophylaxis): Autism Spectrum Disorder, including Autism itself, Asperger's Syndrome, Pervasive Developmental Disorder, and Childhood Disintegrative Disorder. In alternative examples, provided are compositions and methods for treating, ameliorating, reversing and/or preventing (acting as a prophylaxis) Rett Syndrome, Obsessive Compulsive Disorders (OCD), various anxiety disorders as well as major depressive disorders, bipolar disorders, anorexia nervosa, bulimia nervosa, Tourette's syndrome, Attention Deficit Hyperactivity Disorder, Trichotillomania and Dermatillomania.

### Multicomponent Packaging

Provided are multi-component delivery systems, e.g., products of manufacture, comprising e.g., formulations, pharmaceutical preparations or pharmaceutical compositions used to practice methods as provided herein, e.g., formulated and dosaged for oral administration as a powder, e.g., a lyophilised powder, and another component, e.g., a liquid; these multi-component delivery systems, e.g., products of manufacture, can be designed or manufactured as described e.g., in USPNs 8,968,717; 8,931,665; 7,861,854; 7,018,089; 6,626,912; and, U.S. Pat. App. Pub nos. 2010/0034574; 2009/0180923; 20090232886; 2008/0160076; 2007/0087048; 2007/0036830; 2007/0074979; 2005/0205438; 2004/0089563.

### Packaging

Provided are compositions, including preparations, formulations and/or kits, comprising combinations of ingredients, as described herein. In alternative examples, these combinations can be mixed and administered together, or alternatively, they can be an individual member of a packaged combination of ingredients, e.g., a liquid component and a solid product component manufactured in a separate compartment, package, kit or container; e.g., where all or a subset of the combinations of ingredients are manufactured in a separate compartment, package or container. In alternative examples, the package, kit or container comprises a blister package, a clamshell, a tray, a shrink wrap and the like.

In one examples, the package, kit or container comprises a "blister package" (also called a blister pack, or bubble pack). In one example, the blister package is made up of two separate elements: a transparent plastic cavity shaped to the product and its blister board backing. These two elements are then joined together with a heat sealing process which allows the product to be hung or displayed. Exemplary types of "blister packages" include: Face seal blister packages, gang run blister packages, mock blister packages, interactive blister packages, slide blister packages.

Blister packs, clamshells or trays are forms of packaging used for goods; thus, provided are for blister packs, clamshells or trays comprising a formulations, pharmaceutical preparations or pharmaceutical compositions used to practice methods as provided herein. Blister packs, clamshells or trays can be designed to be non-reclosable, so consumers can tell if a package has already opened. They are used to package for sale goods where product tampering is a consideration, such as the pharmaceuticals as provided herein. In one examples, a blister pack comprises a moulded PVC base, with raised areas (the "blisters") to contain the tablets, pills, etc. comprising the combinations of formulations, pharmaceutical preparations or pharmaceutical compositions as provided herein, covered by a foil laminate. Tablets, pills, etc. are removed from the pack either by peeling the foil back or by pushing the blister to force the tablet to break the foil. In one example, a specialized form of a blister pack is a strip pack. In one example, in the United Kingdom, blister packs adhere to British Standard 8404.

In one example, provided is a method of packaging wherein the compositions comprising combinations of ingredients are contained in-between a card and a clear PVC. The PVC can be transparent so the item (pill, tablet, geltab, etc.) can be seen and examined easily; and in one example, can be vacuum-formed around a mould so it can contain the item snugly and have room to be opened upon purchase. In one example, the card is brightly colored and designed depending on the item (pill, tablet, geltab, etc.) inside, and the PVC is affixed to the card using pre-formed tabs where the adhesive is placed. The adhesive can be strong enough so that the pack may hang on a peg, but weak enough so that this way one can tear open the join and access the item. Sometimes with large items or multiple enclosed pills, tablets, geltabs, etc., the card has a perforated window for access. In one example, more secure blister packs, e.g., for items such as pills, tablets, geltabs, etc. are used, and they can comprise of two vacuum-formed PVC sheets meshed together at the edges, with the informative card inside. These can be hard to open by hand, so a pair of scissors or a sharp knife may be required to open.

In one example, blister packaging comprises at least two or three or more components: a thermoformed "blister" which houses multi-ingredient combination as provided herein, and then a "blister card" that is a printed card with an adhesive coating on the front surface. During the assembly process, the blister component, which is most commonly made out of PVC, is attached to the blister card using a blister machine. This machine introduces heat to the flange area of the blister which activates the glue on the card in that specific area and ultimately secures the PVG blister to the printed blister card. The thermoformed PVG blister and the printed blister card can be as small or as large as you would like, but there are limitations and cost considerations in going to an oversized blister card. Conventional blister packs can also be sealed (e.g., using an AERGO 8 DUO^{™}, SCA Consumer Packaging, Inc., DeKalb IL) using regular heat seal tooling. This alternative example, using heat seal tooling, can seal common types of thermoformed packaging.

In alternative examples, formulations, pharmaceutical preparations or pharmaceutical compositions are formulated, e.g., as a powder, e.g., as lyophilised material, e.g., a lyophilized encapsulated product, e.g., for practicing methods as provided herein, can be packaged alone or in combinations, e.g., as "blister packages" or as a plurality of packettes, including as lidded blister packages, lidded blister or blister card or packets or packettes, or a shrink wrap.

In alternative examples, laminated aluminium foil blister packs are used, e.g., for the preparation of formulations, pharmaceutical preparations or pharmaceutical compositions as provided herein. Products or kits comprise an aqueous solution(s) which are dispensed (e.g., by measured dose) into containers. Trays can be freeze-dried to form tablets which take the shape of the blister pockets. The alufoil laminate of both the tray and lid fully protects any highly hygroscopic and/or sensitive individual doses. In one example, the pack incorporates a child-proof peel open security laminate. In one example, the system give tablets an identification mark by embossing a design into the alufoil pocket that is taken up by the tablets when they change from aqueous to solid state. In one example, individual 'push-through' blister packs/ packettes are used, e.g., using hard temper aluminium (e.g., alufoil) lidding material. In one example, hermetically-sealed high barrier aluminium (e.g., alufoil) laminates are used. In one example, products of manufacture include kits or blister packs, use foil laminations and strip packs, stick packs, sachets and pouches, peelable and non-peelable laminations combining foil, paper, or film for high barrier packaging.

In alternative examples, multi-component products of manufacture, including kits or blister packs as provided herein, include memory aids to help remind patients when and how to take the therapeutic agent. This safeguards the therapeutic agent's efficacy by protecting each tablet, geltab or pill until it's taken; gives the product or kit portability, makes it easy to take a dose anytime or anywhere.

### Examples

### Example 1

A 14y old autistic child (averbal) with OCD and severe abdominal pains, gas and constipation, was commenced on Rifaximin 500 mg bid, metronidazole 200 bid later increased to 400 bid, to treat the gastrointestinal symptoms. Progressively his bowel symptoms improved, albeit incompletely and vancomycin 250 mg bid was added. Over the next 6 months his abdominal symptoms completely resolved and he could sleep through the night without being woken by pains. At about 9 months on continuous treatment the parents reported on a Skype consultation in the presence of his two carers that the boy now went to school, could speak more than 800 words, and carried out commands. During the consultation, the child was asked to go to the garage and bring back his father's coat which was on the back seat of the car. The child promptly carried out the request. He continues to be on the oral drugs and attends school.

### Example 2:

A 22y old male patient with ASD unable to say more than 2 word-like noises (mamma and 'no') was brought for treatment mainly because of his aggressive behaviour (requiring two carers holding him in the office) and self-damaging OCD. He hit his head against a brick wall till his scalp was deeply cut to visible bone, and bleeding. He was placed on tinidazole 500 mg bid, rifaximin 500 mg bid increasing to 1 g bid after 2 weeks, and vancomycin 250 mg ii bid. Over the next 6 months the patient had progressively lost his aggression, could sit and watch movies unattended, and had progressively lost most of his OCD symptoms - especially that of chewing a large rubber 'snake' toy. Over the next 6 months the patient learned to speak 3 words. He continues on the medications given the marked improvement in his behaviour and word growth.

## Claims

1. A composition comprising a rifaximin and at least one additional antimicrobial or antibiotic agent, wherein the at least one additional antimicrobial or antibiotic agent comprises a combination of a vancomycin, with a metronidazole, and/or a tinidazole,
for use in treating or preventing autism or autism spectrum disorder (ASD) in an individual in need thereof.

2. The composition for use of claim 1, wherein the autism or the autism spectrum disorder (ASD) is selected from the group consisting of autistic disorder, pervasive developmental disorder not otherwise specified (PDD-NOS), regressive autism setback-type autism, acquired autistic syndrome, and Asperger syndrome.

3. The composition for use of claim 1 or claim 2, wherein the individual exhibits at least an about 5% to 10% reduction in autism or an autism spectrum disorder (ASD) symptom severity after administration of the formulation, pharmaceutical preparation or pharmaceutical composition to the individual in need thereof as compared to before initiating the administration, wherein the reduction in symptom severity is based on an assessment system selected from the group consisting of Childhood Autism Rating Scale (CARS), Childhood Autism Rating Scale 2--Standard Form (CARS2-ST), Childhood Autism Rating Scale 2--High Functioning (CARS2-HF), and a combination thereof.

4. The composition for use of claim 3, wherein the at least about 5% to 10% reduction in autism or an autism spectrum disorder (ASD) symptom severity is achieved after about 1 to 2 or more weeks, or after about 1 to 2 months, of initiating the administration.

5. The composition for use of claim 3, wherein the at least about 5% to 10% reduction in autism or an autism spectrum disorder (ASD) symptom severity is maintained for at least about 4 to 8 weeks after discontinuing the administration.

6. The composition for use of any one of the preceding claims, wherein a unit dosage of the composition is a pediatric unit dosage, and optionally the unit dosage is between about 10 mg and 1100 mg, or is about 10, 20, 30, 40, 50, 60, 70, 75, 80, 90, 100, 125, 150, 175, 200, 225, 250, 275, 300, 325, 350, 375, 400, 425, 450, 475, 500, 600, 700, 750, 800, 900, 1000, 1100 or more mg per unit dose.

7. The composition for use of any one of the preceding claims, wherein a daily dosage of the composition is about 100, 125, 150, 175, 200, 225, 250, 275, 300, 325, 350, 375, 400, 425, 450, 475, 500, 600, 700, 750, 800, 900, 1000 or 1100 or more mg per day, or between about 100 and 1100 mg per day.

8. The composition for use of any one of the preceding claims, wherein a unit dosage of the composition is set for bid (twice a day), tid (three times a day), four times a day, five times a day or six times a day or more, with the unit dosage and daily dosage adjusted to be: about 1000 mg/70 kg a day, or about 14 mg/kg a day, for an adult median dose per day; or for a pediatric dosage about 350 mg/25 kg a day, or about 15 to 16 mg/kg, a day; or equivalent.

9. The composition for use of any one of the preceding claims, wherein the composition further comprises a flavoring or a sweetening agent, aspartame, stevia, monk fruit, sucralose, saccharin, cyclamate, xylitol, vanilla, artificial vanilla or chocolate or strawberry flavor, artificial chocolate essence, or a mixture or combination thereof; and/or
wherein the composition further comprises a preservative, benzoic acid or potassium sorbate.

10. The composition for use of any one of the preceding claims, wherein the composition further comprises at least one probiotic or prebiotic, wherein optionally the prebiotic comprises inulin, lactulose, extracts of artichoke, chicory root, oats, barley, legumes, garlic, kale, beans, flax, or an herb, wherein optionally the probiotic comprises a cultured or stool-extracted microorganism or bacteria, or a bacterial component, and optionally the bacteria or bacterial component comprises or is derived from a *Bacteroidetes*, a *Firmicutes*, a *Lactobacilli*, a *Bifidobacteria,* an *E coli,* a *Strep fecalis* and equivalents.

11. The composition for use of any one of the preceding claims, wherein the composition further comprises at least one congealing agent, wherein optionally the congealing agent comprises arrowroot or plant starch, powdered flour, powdered potato or potato starch, an absorbant polymer, an Absorbable Modified Polymer, and/or corn flour or corn starch.

12. The composition for use of any one of the preceding claims, wherein the composition further comprises an additive selected from one or more of saline, a media, a defoaming agent, a surfactant agent, a lubricant, an acid neutralizer, a marker, a cell marker, a drug, an antibiotic, a contrast agent, a dispersal agent, a buffer or a buffering agent, a sweetening agent, a debittering agent, a flavoring agent, a pH stabilizer, an acidifying agent, a preservative, a desweetening agent a coloring agent, a vitamin, a mineral, a dietary supplement, and a prebiotic nutrient.

13. The composition for use of any one of the preceding claims, wherein the composition further comprises at least one biofilm disrupting compound, wherein optionally the biofilm disrupting compound comprises an enzyme, a deoxyribonuclease (DNase), N-acetylcysteine, auranofin, an alginate lyase, glycoside hydrolase dispersin B, a quorum-sensing inhibitor, a ribonucleic acid III inhibiting peptide, *Salvadora persica* extracts, competence-stimulating peptide, patulin and penicillic acid, peptides, cathelicidin-derived peptides, small lytic peptide, PTP-7, nitric oxide, neo-emulsions, ozone, lytic bacteriophages, lactoferrin, xylitol hydrogel, synthetic iron chelators, cranberry components, curcumin, silver nanoparticles, acetyl-11-keto-β-boswellic acid (AKBA), barley coffee components, probiotics, sinefungin, S-adenosylmethionine, S-adenosyl-homocysteine, *Delisea* furanones, N-sulfonyl homoserine lactones, or any combination thereof.

14. The composition for use of any one of the preceding claims, wherein the composition is formulated as a delayed or gradual enteric release composition, and optionally the composition comprises a gastro-resistant coating designed to dissolve at a pH of 7 in the terminal ileum, and optionally the active ingredients are coated with an acrylic based resin or equivalent, optionally a poly(meth)acrylate, optionally a methacrylic acid copolymer B, NF, which dissolves at pH 7 or greater, and optionally comprises a multimatrix (MMX) formulation.

15. The composition for use of any one of the preceding claims, wherein the composition is formulated as a liquid, a suspension, a gel, a geltab, a semisolid, a tablet, a sachet, a lozenge or a capsule, or as an enteral formulation, or re-formulated for final delivery as a liquid, a suspension, a gel, a geltab, a semisolid, a tablet, a sachet, a lozenge or a capsule, or as an enteral formulation, and/or wherein the composition is formulated as a chewable delivery vehicle, a gum, a gummy, a candy, an ice cream or an ice, or a yogurt.

## Patentansprüche

1. Zusammensetzung, umfassend ein Rifaximin und mindestens einen zusätzlichen antimikrobiellen oder antibiotischen Wirkstoff, wobei der mindestens eine zusätzliche antimikrobielle oder antibiotische Wirkstoff eine Kombination aus einem Vancomycin mit einem Metronidazol und/oder einem Tinidazol umfasst, zur Verwendung bei der Behandlung oder Vorbeugung von Autismus oder Autismus-Spektrum-Störungen (ASS) bei einem Individuum, das dies benötigt.

2. Zusammensetzung zur Verwendung nach Anspruch 1, wobei der Autismus oder die Autismus-Spektrum-Störung (ASS) aus der Gruppe ausgewählt wird, die aus autistischer Störung, nicht näher bezeichneter tiefgreifender Entwicklungsstörung (PDD-NOS), regressivem Autismus und Asperger-Syndrom besteht.

3. Zusammensetzung zur Verwendung nach Anspruch 1 oder 2, wobei das Individuum nach Verabreichung der Formulierung, des pharmazeutischen Präparats oder der pharmazeutischen Zusammensetzung an das Individuum, das diese(s) benötigt, mindestens eine etwa 5 bis 10%ige Verringerung der Schwere der Symptome von Autismus oder einer Autismus-Spektrum-Störung (ASS) im Vergleich zu vor Beginn der Verabreichung aufweist, wobei die Verringerung der Schwere der Symptome auf einem Bewertungssystem basiert, das aus der Gruppe ausgewählt ist, die aus Childhood Autism Rating Scale (CARS), Childhood Autism Rating Scale 2-Standard Form (CARS2-ST), Childhood Autism Rating Scale 2-High Functioning (CARS2- HF) und einer Kombination daraus besteht.

4. Zusammensetzung zur Verwendung nach Anspruch 3, wobei die mindestens etwa 5 bis 10%ige Verringerung der Schwere der Symptome von Autismus oder einer Autismus-Spektrum-Störung (ASS) nach etwa 1 bis 2 oder mehr Wochen oder nach etwa 1 bis 2 Monaten nach Beginn der Verabreichung erreicht wird.

5. Zusammensetzung zur Verwendung nach Anspruch 3, wobei die mindestens etwa 5 bis 10%ige Verringerung der Schwere der Symptome von Autismus oder einer Autismus-Spektrum-Störung (ASS) für mindestens etwa 4 bis 8 Wochen nach Absetzen der Verabreichung aufrecht bleibt.

6. Zusammensetzung zur Verwendung nach einem der vorangehenden Ansprüche, wobei eine Einheitsdosierung der Zusammensetzung eine pädiatrische Einheitsdosierung ist und die Einheitsdosierung optional zwischen etwa 10 mg und 1100 mg liegt oder etwa 10, 20, 30, 40, 50, 60, 70, 75, 80, 90, 100, 125, 150, 175, 200, 225, 250, 275, 300, 325, 350, 375, 400, 425, 450, 475, 500, 600, 700, 750, 800, 900, 1000 oder 1100 oder mehr mg pro Einheitsdosis beträgt.

7. Zusammensetzung zur Verwendung nach einem der vorangehenden Ansprüche, wobei eine tägliche Dosierung der Zusammensetzung etwa 100, 125, 150, 175, 200, 225, 250, 275, 300, 325, 350, 375, 400, 425, 450, 475, 500, 600, 700, 750, 800, 900, 1000 oder 1100 oder mehr mg pro Tag oder zwischen etwa 100 und 1100 mg pro Tag beträgt.

8. Zusammensetzung zur Verwendung nach einem der vorangehenden Ansprüche, wobei eine Einheitsdosierung der Zusammensetzung auf zweimal täglich (bid), dreimal täglich (tid), viermal täglich, fünfmal täglich oder sechsmal täglich oder öfter eingestellt wird, wobei die Einheitsdosierung und die tägliche Dosierung wie folgt angepasst werden: etwa 1000 mg/70 kg täglich oder etwa 14 mg/kg täglich für eine mittlere Tagesdosis für Erwachsene; oder für eine pädiatrische Dosierung etwa 350 mg/25 kg täglich oder etwa 15 bis 16 mg/kg täglich; oder ein Äquivalent.

9. Zusammensetzung zur Verwendung nach einem der vorangehenden Ansprüche, wobei die Zusammensetzung ferner einen Geschmacksstoff oder ein Süßungsmittel, Aspartam, Stevia, Mönchsfrucht, Sucralose, Saccharin, Cyclamat, Xylit, Vanille, künstliches Vanille-, Schokoladen- oder Erdbeeraroma, künstliche Schokoladenessenz oder eine Mischung oder Kombination daraus umfasst; und/oder
wobei die Zusammensetzung ferner ein Konservierungsmittel, Benzoesäure oder Kaliumsorbat umfasst.

10. Zusammensetzung zur Verwendung nach einem der vorangehenden Ansprüche, wobei die Zusammensetzung ferner mindestens ein Probiotikum oder Präbiotikum umfasst, wobei das Präbiotikum optional Inulin, Lactulose, Extrakte aus Artischocke, Zichorienwurzel, Hafer, Gerste, Hülsenfrüchten, Knoblauch, Kohl, Bohnen, Flachs oder einem Kraut umfasst, wobei das Probiotikum optional eine(n) kultivierte(n) oder aus dem Stuhl extrahierte(n) Mikroorganismus oder Bakterie oder eine bakterielle Komponente umfasst und wobei die Bakterie oder die bakterielle Komponente optional ein *Bacteroidetes,* ein *Firmicutes,* ein *Lactobacillus,* ein *Bifidobacterium,* ein *E. coli,* ein *Strep. faecalis* und Äquivalente umfasst oder daraus abgeleitet ist.

11. Zusammensetzung zur Verwendung nach einem der vorangehenden Ansprüche, wobei die Zusammensetzung ferner mindestens ein Verfestigungsmittel umfasst, wobei das Verfestigungsmittel optional Pfeilwurzel oder Pflanzenstärke, Mehlpulver, Kartoffelpulver oder Kartoffelstärke, ein absorbierendes Polymer, ein Absorbable Modified Polymer und/oder Maismehl oder Maisstärke umfasst.

12. Zusammensetzung zur Verwendung nach einem der vorangehenden Ansprüche, wobei die Zusammensetzung ferner einen Zusatzstoff umfasst, ausgewählt aus einem oder mehreren aus einer Kochsalzlösung, einem Medium, einem Entschäumungsmittel, einem oberflächenaktiven Stoff, einem Schmiermittel, einem Säureneutralisator, einem Marker, einem Zellmarker, einem Arzneimittel, einem Antibiotikum, einem Kontrastmittel, einem Dispersionsmittel, einem Puffer oder einem Puffermittel, einem Süßungsmittel, einem Entbitterungsmittel, einem Geschmacksmittel, einem pH-Stabilisator, einem Säuerungsmittel, einem Konservierungsmittel, einem Entsüßungsmittel, einem Farbstoff, einem Vitamin, einem Mineral, einem Nahrungsergänzungsmittel und einem präbiotischen Nährstoff.

13. Zusammensetzung zur Verwendung nach einem der vorangehenden Ansprüche, wobei die Zusammensetzung ferner mindestens eine Biofilm-zerstörende Verbindung umfasst, wobei die Biofilm-zerstörende Verbindung optional ein Enzym, eine Desoxyribonuklease (DNase), N-Acetylcystein, Auranofin, eine Alginatlyase, Glykosidhydrolase Dispersin B, einen Quorum-Sensing-Inhibitor, ein Ribonukleinsäure-III-inhibierendes Peptid, Extrakte aus *Salvadora persica,* Kompetenzstimulierendes Peptid, Patulin und Penicillinsäure, Peptide, von Cathelicidin abgeleitete Peptide, kleines lytisches Peptid, PTP-7, Stickstoffmonoxid, Neoemulsionen, Ozon, lytische Bakteriophagen, Lactoferrin, Xylit-Hydrogel, synthetische Eisenchelatoren, Cranberry-Bestandteile, Curcumin, Silbernanopartikel, Acetyl-11-keto-β-Boswelliasäure (AKBA), Gerstenkaffee-Bestandteile, Probiotika, Sinefungin, S-Adenosylmethionin, S-Adenosylhomocystein, *Delisea*-Furanone, N-Sulfonylhomoserinlactone oder eine beliebige Kombination davon umfasst.

14. Zusammensetzung zur Verwendung nach einem der vorangehenden Ansprüche, wobei die Zusammensetzung als eine Zusammensetzung mit verzögerter oder allmählicher Freisetzung im Darm formuliert ist und die Formulierung optional eine magensaftresistente Beschichtung umfasst, die dazu bestimmt ist, sich bei einem pH-Wert von 7 im terminalen Ileum aufzulösen, und die Wirkstoffe optional mit einem auf Acryl basierenden Harz oder einem Äquivalent, optional mit einem Polymethacrylat, optional mit einem Methacrylsäure-Copolymer B, NF, das sich bei einem pH-Wert von 7 oder höher auflöst, beschichtet sind und optional eine Multimatrix-Formulierung (MMX) umfasst.

15. Zusammensetzung zur Verwendung nach einem der vorangehenden Ansprüche, wobei die Zusammensetzung als eine Flüssigkeit, eine Suspension, ein Gel, eine Geltablette, ein Halbfeststoff, eine Tablette, ein Beutel, eine Lutschtablette oder eine Kapsel oder als eine enterale Formulierung formuliert ist oder für die endgültige Verabreichung als eine Flüssigkeit, eine Suspension, ein Gel, eine Geltablette, ein Halbfeststoff, eine Tablette, ein Beutel, eine Lutschtablette oder eine Kapsel oder als eine enterale Formulierung neu formuliert wird und/oder wobei die Zusammensetzung als eine kaubare Verabreichungsform, ein Kaugummi, ein Gummibonbon, ein Bonbon, eine Eiscreme oder ein Eis oder ein Joghurt formuliert ist.

## Revendications

1. Composition comprenant une rifaximine et au moins un agent antimicrobien ou antibiotique supplémentaire, dans laquelle l'au moins un agent antimicrobien ou agent antibiotique supplémentaire comprend une combinaison d'une vancomycine, avec un métronidazole et/ou un tinidazole, à utiliser dans le traitement ou la prévention de l'autisme ou d'un trouble du spectre autistique (ASD) chez un individu en ayant besoin.

2. Composition à utiliser selon la revendication 1, dans laquelle l'autisme ou le trouble du spectre autistique (ASD) est sélectionné à partir du groupe composé du trouble autistique, le trouble envahissant du développement non spécifié (TED-NS), l'autisme de type régressif, le syndrome autistique acquis et le syndrome d'Asperger.

3. Composition à utiliser selon la revendication 1 ou la revendication 2, dans laquelle l'individu manifeste une réduction d'au moins environ 5 % à 10 % de la gravité des symptômes de l'autisme ou d'un trouble du spectre autistique (ASD) après l'administration de la formulation, de la préparation pharmaceutique ou de la composition pharmaceutique à l'individu qui en a besoin par rapport à avant le début de l'administration, dans laquelle la réduction de la gravité des symptômes étant basée sur un système d'évaluation sélectionné dans le groupe composé de l'échelle d'évaluation de l'autisme infantile (CARS), l'échelle d'évaluation de l'autisme infantile 2--forme standard (CARS2-ST), l'échelle d'évaluation de l'autisme infantile 2--fonction élevée (CARS2-HF), et d'une combinaison de celles-ci.

4. Composition à utiliser selon la revendication 3, dans laquelle une réduction d'au moins environ 5 % à 10 % de la gravité des symptômes de l'autisme ou d'un trouble du spectre autistique (ASD) soit obtenue après environ 1 à 2 semaines ou plus, ou après environ 1 à 2 mois, après le début de l'administration.

5. Composition à utiliser selon la revendication 3, dans laquelle la réduction d'au moins environ 5 % à 10 % de la gravité des symptômes d'un autisme ou d'un trouble du spectre autistique (ASD) est maintenue pendant au moins environ 4 à 8 semaines après l'arrêt de l'administration.

6. Composition à utiliser selon l'une quelconque des revendications précédentes, dans laquelle une dose unitaire de la composition est une dose unitaire pédiatrique, et éventuellement la dose unitaire est comprise entre environ 10 mg et 1100 mg, ou est d'environ 10, 20, 30, 40, 50, 60, 70, 75, 80, 90, 100, 125, 150, 175, 200, 225, 250, 275, 300, 325, 350, 375, 400, 425, 450, 475, 500, 600, 700, 750, 800, 900, 1000, 1100 mg ou plus par dose unitaire.

7. Composition à usage selon l'une quelconque des revendications précédentes, dans laquelle la dose quotidienne de la composition est d'environ 100, 125, 150, 175, 200, 225, 250, 275, 300, 325, 350, 375, 400, 425, 450, 475, 500, 600, 700, 750, 800, 900, 1000 ou 1100 mg ou plus par jour, ou entre environ 100 et 1100 mg par jour.

8. Composition à usage selon l'une quelconque des revendications précédentes, dans laquelle une dose unitaire de la composition est fixée à bid (deux fois par jour), tid (trois fois par jour), quatre fois par jour, cinq fois par jour ou six fois par jour ou plus, dans laquelle la dose unitaire et la dose quotidienne soient ajustées à : environ 1000 mg/70 kg par jour, ou environ 14 mg/kg par jour, pour une dose médiane adulte par jour ; ou pour une dose pédiatrique d'environ 350 mg/25 kg par jour, ou environ 15 à 16 mg/kg par jour ; ou équivalent.

9. Composition à utiliser selon l'une quelconque des revendications précédentes, dans laquelle la composition comprend en outre un agent aromatisant ou un agent édulcorant, l'aspartame, la stevia, le fruit de moine, du sucralose, la saccharine, le cyclamate, le xylitol, la vanille, un arôme artificiel de vanille ou de chocolat ou de fraise, une essence artificielle de chocolat, ou un mélange ou une combinaison de ceux-ci ; et/ou
dans laquelle la composition comprend en outre un conservateur, l'acide benzoïque ou le sorbate de potassium.

10. Composition à utiliser selon l'une quelconque des revendications précédentes, dans laquelle la composition comprend en outre au moins un probiotique ou un prébiotique, dans laquelle éventuellement le prébiotique comprend l'inuline, le lactulose, des extraits d'artichaut, de racine de chicorée, d'avoine, d'orge, de légumineuses, d'ail, de chou frisé, d'haricots, de lin ou d'une herbe, dans laquelle éventuellement le probiotique comprend un micro-organisme ou une bactérie cultivés ou extraits de selles, ou un composant bactérien, et éventuellement la bactérie ou le composant bactérien comprend ou est dérivé d'un *Bacteroidetes,* d'un *Firmicutes,* d'un *Lactobacilli,* d'un *Bifidobacteria,* d'un *E coli,* d'un *Strep fecalis* et équivalents.

11. Composition à utiliser selon l'une quelconque des revendications précédentes, dans laquelle la composition comprend en outre au moins un agent de coagulation, dans laquelle éventuellement l'agent de coagulation comprend la marante ou l'amidon végétal, la farine en poudre, la pomme de terre en poudre ou l'amidon de pomme de terre, un polymère absorbant, un polymère modifié absorbable et/ou la farine de maïs ou l'amidon de maïs.

12. Composition à utiliser selon l'une quelconque des revendications précédentes, dans laquelle la composition comprend en outre un additif sélectionné parmi un ou plusieurs éléments parmi une solution saline, un milieu, un agent antimousse, un agent tensioactif, un lubrifiant, un neutralisant d'acide, un marqueur, un marqueur cellulaire, un médicament, un antibiotique, un agent de contraste, un agent de dispersion, un tampon ou un agent tampon, un agent édulcorant, un agent désamérisant, un agent aromatisant, un stabilisateur de pH, un agent acidifiant, un conservateur, un agent désédulcorant, un colorant, une vitamine, un minéral, un complément alimentaire et un nutriment prébiotique.

13. Composition à utiliser selon l'une quelconque des revendications précédentes, dans laquelle la composition comprend en outre au moins un composé perturbateur de biofilm, dans laquelle éventuellement le composé perturbateur de biofilm comprend une enzyme, une désoxyribonucléase (DNase), la N-acétylcystéine, l'auranofine, une alginate lyase, la glycoside hydrolase dispersine B, un inhibiteur de détection de quorum, un peptide inhibiteur de l'acide ribonucléique III, des extraits de *Salvadora persica,* un peptide stimulant la compétence, la patuline et l'acide pénicillique, des peptides, des peptides dérivés de la cathélicidine, le petit peptide lytique, PTP-7, l'oxyde nitrique, des néoémulsions, l'ozone, des bactériophages lytiques, la lactoferrine, un hydrogel de xylitol, des chélateurs de fer synthétiques, des composants de canneberge, la curcumine, des nanoparticules d'argent, de l'acide acétyl-11-céto-β-boswellique (AKBA), des composants de café d'orge, des probiotiques, le sinefungine, le S-adénosylméthionine, le S-adénosyl-homocystéine, des furanones de *Delisea,* des lactones de N-sulfonyle homosérine, ou toute combinaison de ceux-ci.

14. Composition à utiliser selon l'une quelconque des revendications précédentes, dans laquelle la composition est formulée sous forme de composition à libération entérique retardée ou progressive, et éventuellement la composition comprend un enrobage gastro-résistant conçu pour se dissoudre à un pH de 7 dans l'iléon terminal, et éventuellement les ingrédients actifs sont enrobés d'une résine à base d'acrylique ou équivalent, éventuellement d'un poly(méth)acrylate, éventuellement d'un copolymère d'acide méthacrylique B, NF, qui se dissout à un pH de 7 ou plus, et comprend éventuellement une formulation multimatrice (MMX).

15. Composition à utiliser selon l'une quelconque des revendications précédentes, dans laquelle la composition est formulée sous forme de liquide, de suspension, de gel, de comprimé gélifié, de semi-solide, de comprimé, de sachet, de pastille ou de capsule, ou sous forme de formulation entérale, ou reformulée pour une administration finale sous forme de liquide, de suspension, de gel, de comprimé gélifié, de semi-solide, de comprimé, de sachet, de pastille ou de capsule, ou sous forme de formulation entérale, et/ou dans laquelle la composition est formulée sous forme de véhicule d'administration à croquer, de gomme, de bonbon gélifié, de crème glacée ou de glace, ou de yaourt.
